# EUROPEAN PATENT APPLICATION

(11) **EP 2 184 036 A1**
(43) Date of publication of application: **12.05.2010**
(21) Application number: 08019607.4
(22) Date of filing: 10.11.2008
(51) Int. Cl.: A61F 2/18, A61F 5/08, A61F 5/56

(54) **Nose implant**

(71) Applicant: Seyrek, Göksel, 36043 Fulda (DE)
(72) Inventor: Seyrek, Göksel, 36043 Fulda (DE)
(74) Representative: Tergau & Pohl Patentanwälte

(57) **Abstract**

A nose implant (1), in particular for insertion into the respiratory channel of a human nose, including a channel body (2) forming a breathing-air channel, shall reduce a contact of foreign matter with the nasal mucous membrane, without creating at the same time a perceptible impairment of the nasal functions, in particular breathing in and breathing out. For this purpose, a sealing element (4) acting in the manner of a check valve is provided in the channel body (2).

## Description

The invention relates to a nose implant, in particular for insertion into the respiratory channel of a human nose.

The invention is based on the task to provide a device, in particular a nose implant of the above-mentioned type, which reduces a contact of foreign matter with the nasal mucous membrane, without creating at the same time a perceptible impairment of the nasal functions, in particular breathing in and breathing out.

This task is solved by the invention in that the nose implant includes a channel body forming a breathing-air channel, in which a sealing element acting in the manner of a check valve is arranged.

The invention is based on the idea that a contact of foreign matter with the nasal mucous membrane mainly occurs through intense or jerky breathing-in of the foreign matter, the foreign matter being transported in the suction of the air flow through the respiratory channel of the nose. Therefore, to avoid the contact of this foreign matter with the nasal mucous membrane, the strong air flow caused by the jerky breathing-in is interrupted. For such an interruption of the air flow in one direction, namely in the direction of the nasal mucous membrane, a check valve is used. For this purpose, a sealing element acting in the manner of a check valve is arranged in the breathing-air channel of a nose implant.

To offer a particularly high wearing comfort, the nose implant includes, in an advantageous embodiment, a channel body having a tapered inner cross-section, the cross-section of a nose implant tapering, in implanted condition, in the direction of the nasal mucous membrane. In this way, a secure fit of the nose implant can be achieved, which is decisive for the wearing comfort and an unhampered breathing in and out. Through the funnel-shaped design of the nose implant, a particularly good ergonomic adaptation to the respiratory channel of the human nose is achieved.

To secure a particularly good interruption of the air flow in case of jerky breathing-in and to avoid in this way the contact of foreign matter with the nasal mucous membrane, the sealing element is formed, in an advantageous embodiment, as a mobile body. This mobile body is designed in such a manner that it is moved, in case of jerky breathing-in, by the air flow generated thereby, in the direction of the nasal mucous membrane and closes the mucous-membrane-side exit of the nose implant. Due to the normally conical embodiment of the nose implant, the mobile body is advantageously a ball. Due to its circular cross-section, such a ball can close the cross-section, which in most cases is also circular, of the mucous-membrane-side exit of the nose implant with particularly high security. To limit a free moving of the mobile body in the nose implant and to achieve a specific control of the mobile body in the direction of the mucous-membrane-side exit of the nose implant, a guide channel is provided in the channel body. Through the air flow occurring with jerky breathing-in, the mobile body situated in this guide channel is specifically driven in the direction of the mucous-membrane-side exit of the nose implant and thus protects the nasal mucous membrane in a particularly reliable way against the penetration of foreign substances.

In an alternative preferable embodiment, the sealing element is formed of a plurality of check valves, these check valves being arranged, during normal breathing in and out through the nose implant, in such a way that the air flow can substantially pass them unhampered, whereas, in case of jerkily breathing in, the check valves are pushed by the occurring air flow in the direction of the mucous-membrane-side exit, overlapping each other such that the air flow and the foreign matter contained therein cannot escape from the nose implant on the mucous-membrane side.

In a particularly preferable embodiment, the base material of the nose implant and also of the sealing elements is made of silicone or titanium. It is a particular advantage to cover the mobile body at least with a silicone layer, as otherwise, an undesired and disturbing chatter may occur, if both the inside of the nose implant and the mobile body are completely made of titanium. Furthermore, the materials silicone and titanium distinguish themselves in particular by body tolerance.

The advantages achieved by the invention consist in particular in that the protection of the nasal mucous membrane against foreign substances can be achieved in a most simple manner. In this connection, it is of particular importance that a passive protection exists, i.e. the wearer of such a nose implant cannot and need not actively control or activate the nose implant. Furthermore, due to its application and functionality, the nose implant cannot be deactivated or manipulated by the wearer. Thus, a particularly reliable protection is achieved.

An exemplary embodiment of the invention is explained in detail by means of a drawing, in which
- FIG. 1: shows a nose implant with a mobile body,
- FIG. 2: shows a closed nose implant with a mobile body,
- FIG. 3: shows a nose implant with a guide channel,
- FIG. 4: shows a nose implant with an alternative sealing element, and
- FIG. 5: shows a nose implant with check valves.

Identical parts are marked with the same reference numbers in all figures.

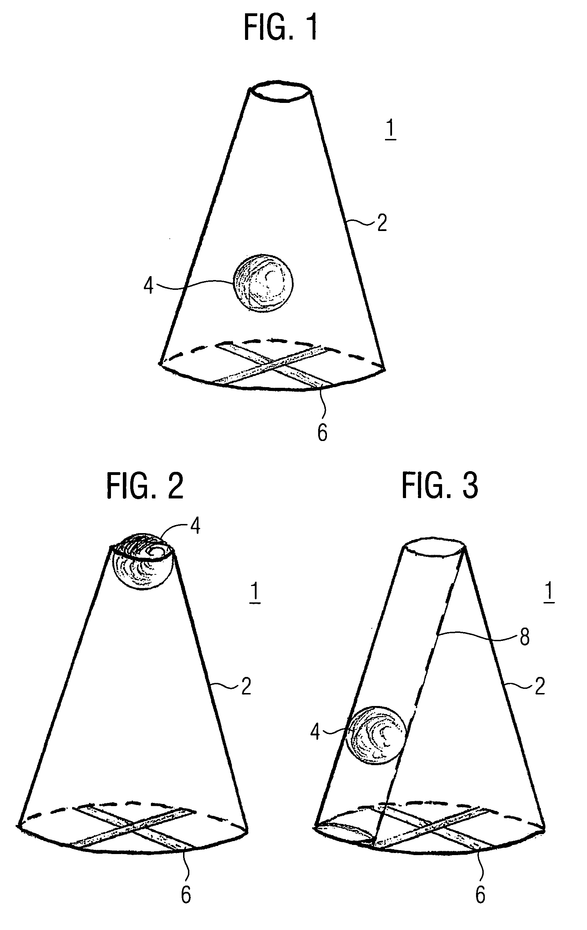

The nose implant 1 according to FIG. 1 comprises a conical channel body 2. Such a nose implant can be implanted into a wing of the nose by a physician in a minor ambulatory operation. The channel body is adapted to the shape of a typical respiratory channel of a wing of the nose, both in its dimensioning and in its design.

Furthermore, however, individual nose implants are imaginable, which are specifically adapted to the patient or to the shape of the respiratory channels of the wings of his/her nose. In conformity with the typical shape of the respiratory channel of a wing of the nose, the cross-section of the channel body tapers, in implanted condition, in the direction of the nasal mucous membrane. In this way, the channel body is adapted in a most simple manner to the anatomical conditions in the area of the nose. This results in a particularly secure fit of the nose implant in the wing of the nose and, at the same time, in the fact that the wearer of the nose implant will hardly notice it and that it will not be visible from outside, for example through a deformation of the wing of the nose.

The sealing element 4 in the nose implant 1 according to FIG. 1 is designed as a ball. However, any mobile body which is able to securely and reliably close the mucous-membrane-side opening of the channel body is imaginable. Therefore, the sealing element 4 in the nose implant 1 could also be of egg-shaped, cylindrical or similar design. The end opposite the mucous-membrane-side end of the channel body 2 is provided with a bottom element 6. The bottom element 6 according to FIG. 1 consists of a plurality of braces (for the sake of simplicity, only two of them are represented) arranged in the form of a cross or a grid. These braces shall prevent a falling-out of the mobile sealing element. At the same time, however, the bottom element 6 must not substantially hamper the air flow during breathing in or out, so that the nose implant 1 will not be described as disturbing or hampering respiration. Therefore, under these secondary conditions, bottom elements 6 of different designs are also imaginable.

The functional state of the nose implant 1 during jerky breathing-in through the nose is shown in FIG. 2. While during normal, calm movements of breathing in and out through the nose, the sealing element 4, due to gravity, preferably moves freely in the area of the bottom element, it is moved, upon jerky breathing-in, due to the stronger air flow, in the direction of the mucous-membrane-side exit of the channel body 2. Diameter and cross-section of the sealing element 4 are adapted to the cross-section of the mucous-membrane-side opening of the channel body 2 in such a way that the sealing element 4 closes that opening upon jerky breathing-in through the nose, so that the air flow in the direction of the nasal mucous membrane is immediately interrupted by the sealing element 4, before any foreign substances present in the air flow can reach the nasal mucous membrane.

This enables a wide range of applications of the nose implant. For example, the nose implant can be helpful in drug therapies. In particular in case of dependence on drugs taken nasally, such as, for example, cocaine, amphetamines, Crystal, such a nose implant can protect its wearer against taking such substances again.

A physician in charge implants one nose implant into each of the patient's wings of the nose, in most cases by an ambulatory treatment and only in exceptional cases by an in-patient treatment. After that, the patient is no longer in a position to nasally take the above-described substances, which can avoid both a relapse during drug disaccustoming and a further taking of the drugs in the addiction stage.

The nose implant 1 according to FIG. 3 additionally comprises a guide channel 8. Through this guide channel 8, the sealing element 4 located therein is specifically guided in the direction of the mucous-membrane-side exit of the channel body 2. The walls of the guide channel 8 must be porous, in particular air-permeable, so that the sealing element 4 is guided by the air flow generated by jerky breathing-in through the nose, in the direction of the mucous-membrane-side exit. The guide channel 8, too, has at the end opposite the mucous-membrane-side end of the channel body 2 a safety device against falling out of the sealing element, similar to the bottom element 6. This guide channel restricts a free movement of the sealing element 4 to the greatest extent possible, so that the channel body can be closed more specifically and more quickly.

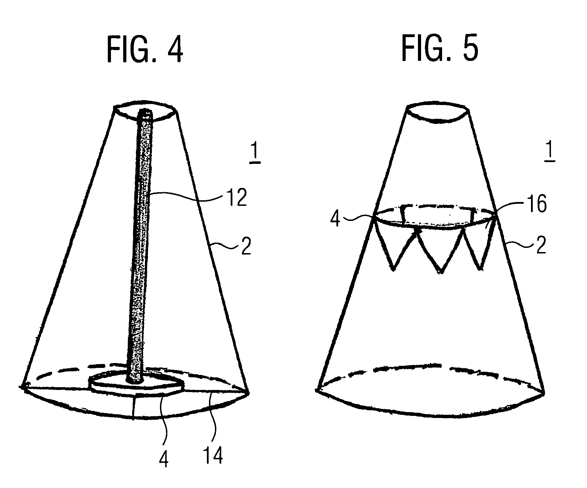

In the nose implant 1 according to FIG. 4, the sealing element 4 is designed in the form of a closing plate 10. This closing plate 10 is also pushed by the air flow generated by jerky breathing-in through the nose in the direction of the mucous-membrane-side opening of the channel body 2, along a guide web 12. This guide web 12 is connected in one or more places with the channel body 2 via positioning elements 14. These are preferably fixed near the mucous-membrane-side end of the channel body 2 or at the end of the channel body 2 opposite thereto, in order not the impair the movement of the closing plate 10 along the guide web 12. The cross-sectional area of the closing plate 10 is adapted to the cross-sectional area of the mucous-membrane-side opening of the channel body 2 or is a little larger in order to safely close the opening. In this way, a secure closing and thus a protection of the nasal mucous membrane against foreign substances upon jerky breathing-in is achieved.

In the nose implant 1 according to FIG. 5, the sealing element 4 is designed in the form of several check valves 16. These check valves 16 are foldably fastened inside the channel body 2 in such a way that in case of normal breathing in and out through the nose, the air flow is guided substantially unhampered through the nose implant 1, whereas in case of jerky breathing-in, the check valves are moved by the air flow in the direction of the mucous-membrane-side exit of the channel body 2, the individual check valves overlapping each other in such a way that the cross-sectional area of the channel body 2 is completely covered and the air flow is in that way interrupted. With this type of embodiment of the sealing element, it can also be achieved in a simple manner that the nasal mucous membrane is protected against contact with foreign substances upon jerky breathing-in through the nose.

Furthermore, when applying the nose implant, it turned out unexpectedly that the wearers of such a nose implant snore considerably less during their sleeping phase, due to a change of the respiratory channels. Therefore, the nose implant can also be applied, in a double function, in addition to the above-described fields of application, for preventing snoring.

List of reference numbers
- 1: Nose implant
- 2: Channel body
- 4: Sealing element
- 6: Bottom element
- 8: Guide channel
- 12: Guide web
- 14: Positioning elements
- 16: Check valves

## Claims

1. Nose implant (1), in particular for insertion into the respiratory channel of a human nose, including a channel body (2) forming a breathing-air channel, a sealing element (4) acting in the manner of a check valve being arranged in said channel body.

2. Nose implant (1) according to claim 1, whose channel body (2) has a tapered inner cross-section.

3. Nose implant (1) according to any of claims 1 to 2, whose sealing element (4) is a mobile body, preferably a ball.

4. Nose implant (1) according to claim 3, in whose channel body a guide channel (8) for the mobile body is provided.

5. Nose implant (1) according to claim 1 or 2, whose sealing element (4) consists of a plurality of check valves (16).

6. Nose implant (1) according to any of claims 1 to 5, whose basic material is silicone or titanium.
